(19) Europäisches Patentamt
European Patent Office
Office européen des brevets

(11) Veröffentlichungsnummer: **0 275 489 B1**

# (12) EUROPÄISCHE PATENTSCHRIFT

(45) Veröffentlichungstag der Patentschrift: **09.08.89**

(51) Int Cl.⁴: **C07C 47/575**, C07C 43/315, C25B 3/02, A61K 7/46

(21) Anmeldenummer: **87118609.4**

(22) Anmeldetag: **15.12.87**

## (54) Neue Benzaldehydderivate, ihre Herstellung und Verwendung.

(30) Priorität: **23.12.86 DE 3644076**

(43) Veröffentlichungstag der Anmeldung **27.07.88 Patentblatt 88/30**

(45) Bekanntmachung des Hinweises auf die Patenterteilung: **09.08.89 Patentblatt 89/32**

(84) Benannte Vertragsstaaten: **BE CH DE ES FR GB IT LI NL**

(56) Entgegenhaltungen:
**EP-A- 0 025 883**
**DE-A- 2 848 397**
**DE-A- 2 912 058**
**DE-A- 3 214 658**
**BEILSTEINS HANDBUCH DER ORGANISCHEN CHEMIE, Band 8, Teil 1, 4. Auflage, 3. Ergänzungswerk, Seiten 372-373, Springer Verlag, 1969, Berlin**

(73) Patentinhaber: **BASF Aktiengesellschaft**, Carl-Bosch-Strasse 38, D-6700 Ludwigshafen(DE)

(72) Erfinder: **Degner, Dieter, Dr., Kurpfalzstrasse 8, D-6701 Dannstadt-Schauernheim(DE)**
Erfinder: **Gramlich, Walter, Dr., Auf der Hoehe 11, D-6803 Edingen-Neckarhausen(DE)**
Erfinder: **Hannebaum, Heinz, Ostring 56, D-6700 Ludwigshafen(DE)**

EP 0 275 489 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Benzaldehydderivate, ihre Herstellung und Verwendung als Riechstoffe oder Riechstoffvorprodukte.

Bekanntlich haben in 4-Stellung substituierte Benzaldehyde als Riechstoffe Verwendung gefunden. So weist z.B. p-Methylbenzaldehyd (p-Tolylaldehyd) einen süß-herben, Bittermandel-artigen Geruch auf. 4-Methoxibenzaldehyd (Anisaldehyd) besitzt dagegen einen intensiv süß-blumigen etwas Heu-ähnlichen Duft. 4-Isopropylbenzaldehyd (Cuminaldehyd) hat einen starken fast unangenehmen grün-herben Geruch.

Es wurde nun gefunden, daß die neuen in 4-Stellung durch eine Alkoxialkylgruppe substituierten Benzaldehydderivate der Formel

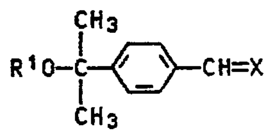

(I),

in der X für ein Sauerstoffatom oder für zwei $R^2O$-Gruppen steht, und $R^1$ und $R^2$ Alkylgruppen mit 1 bis 4 C-Atomen bedeuten, wertvolle Riechstoffe oder Riechstoffvorprodukte darstellen.

Die neuen Benzaldehydderivate dieser Erfindung sind z.B. 4-(1-Alkoxy-1-methylethyl)-benzaldehyde der Formel

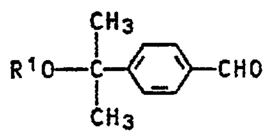

(II)

oder 4-(1-Alkoxy-1-methyl-ethyl)-benzaldehyddialkylacetale der Formel

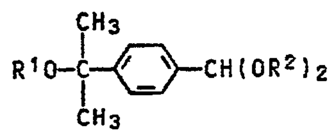

(III),

in denen die Reste $R^1$ und $R^2$ Aklylgruppen mit 1 bis 4 C-Atomen bedeuten.

Die Aldehyde der Formel II haben z.B. einen Geruch, der sich durch eine sehr feine trockene, süßlichholzige Note ausweist, die klar von der herben Note der bisher bekannten in 4-Stellung substituierten Benzaldehyde abweicht.

In den Formeln I bis III stehen die Reste $R^1$ und $R^2$ z.B. für Methyl, Ethyl, Propyl, Isopropyl. Im einzelnen seien die folgenden neuen Benzaldehydderivate genannt: 4-(1-Methoxi-1-methyl-ethyl)-benzaldehyddimethylacetal, 4-(1-Methoxi-1-methyl-ethyl)-benzaldehyd, 4-(1-Ethoxi-1-methyl-ethyl)-benzaldehyd)diethylacetal, 4-(1-Ethoxi-1-methyl-ethyl)-benzaldehyd.

Die neuen Acetale der Formel III lassen sich nach einer besonders eleganten Ausführungsform der Erfindung dadurch herstellen, daß man Benzolderivate der Formel

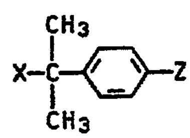

(IV),

in der X ein Wasserstoffatom oder die $R^1O$-Gruppe, Z eine der Gruppen $-CH_3$, $-CH_2OR^2$, $-CH(OR^2)_2$ und $R^1$ und $R^2$ Alkylgruppen mit 1 bis 4 C-Atomen bedeuten, wobei X für ein Wasserstoffatom steht, wenn Z die Gruppe $-CH(OR^2)_2$ bedeutet, in Gegenwart eines Alkanols der Formel ROH, in der R einen Alkylrest mit 1 bis 4 C-Atomen bedeutet, elektrochemisch oxidiert.

Als Ausgangsstoffe für die Herstellung der neuen Acetale der Formel III seien z.B. 4-Isopropyltoluol, 4-(1-Methoxi-1-methyl-ethyl)-toluol, 4-(1-Ethoxi-1-methyl-ethyl)-toluol, 4-Isopropylbenzaldehyddimethylacetal und 4-Isopropylbenzaldehyddiethylacetal genannt. Als Alkanole verwendet man bevorzugt Methanol und Ethanol.

Das erfindungsgemäße Verfahren benötigt keine besondere Zellkonstruktion, es wird bevorzugt in einer ungeteilten Durchflußzelle durchgeführt. Als Anoden kommen z. B. Edelmetallelektroden, wie Pt oder Oxidelektroden wie $Ti/RuO_2$ in Betracht. Bevorzugtes Anodenmaterial ist Graphit. Als Kathoden

kommen z. B. Stahl, Eisen, Nickel, Kupfer, Zink und Kohle sowie Edelmetalle wie Pt in Betracht. Bevorzugtes Kathodenmaterial ist Graphit. Der Elektrolyt setzt sich aus den Verbindungen der Formel IV, dem Alkanol ROH und einem Leitsalz zusammen. Als Leitsalze kommen Neutralsalze, Säuren und Basen in Betracht. Beispiele für Neutralsalze sind Fluoride, wie KF, Sulfonate, wie $NaSO_3C_6H_5$, Sulfate wie $(CH_3)_4NSO_4CH_3$, Tetrafluorborate, wie $NaBF_4$, Phosphate und Phosphonate. Beispiele für Säuren sind Schwefelsäure, Alkyl- und Arylsulfonsäuren, als Basen dienen z.B. Alkoholate, wie $NaOCH_3$ oder Hydroxide, wie KOH.

Der Elektrolyt hat beispielsweise folgende Zusammensetzung 3 bis 60 Gew.% Verbindung der Formel IV
35 bis 90 Gew.% ROH
0,5 bis 10 Gew.% Leitsalz.

Die Stromdichte kann bei dem erfindungsgemäßen Verfahren in weiten Grenzen, z.B. von 0,5 bis 20 A/dm² gewählt werden. Bevorzugt wird bei 1 bis 8 A/dm² gearbeitet. Die Temperaturen bei der Elektrolyse betragen z.B. 20 bis 60°C. Die Elektrolyse wird bevorzugt bei Normaldruck durchgeführt. Die Ausgangsstoffe der Formel IV lassen sich weitgehend umsetzen. Unumgesetzte Zwischenstufen als auch unumgesetztes IV können zur Elektrolyse zurückgeführt werden. Die Elektrolyse kann sowohl diskontinuierlich als kontinuierlich durchgeführt werden.

Die Aufarbeitung der Elektrolyseausträge erfolgt nach konventionellen Methoden. Reagiert der Elektrolyseaustrag sauer, so wird er zunächst mit Basen, wie $NaOCH_3$ neutralisiert. Überschüssiges Alkanol wird abdestilliert. Das Leitsalz wird abfiltriert und kann zusammen mit dem Alkanol bei Bedarf zur Elektrolyse rückgeführt werden. Die Rohacetale können z. B. durch Rektifikation weiter gereinigt werden.

Die neuen Benzaldehyde der Formel II lassen sich sehr vorteilhaft auf an sich bekannte Weise durch Hydrolyse der Acetale der Formel III herstellen, z.B. durch Erhitzen mit Wasser unter Rückfluß.

Die Hydrolyse kann sowohl diskontinuierlich als auch kontinuierlich durchgeführt werden. Der dabei entstehende Alkohol ROH kann auf vorteilhafte Weise für die Herstellung der Acetale der Formel III wieder verwendet werden. Die Abtrennung der neuen Benzaldehyde aus dem Reaktionsgemisch kann dann durch Phasentrennung erfolgen, wenn man bei der Hydrolyse einen Überschuß von Wasser einsetzt. Sofern gewünscht, lassen sich die neuen Benzaldehyde durch Destillation weiter reinigen.

Die Benzaldehyde der Formel II verwendet man aufgrund der obengenannten Eigenschaften als Riechstoffe oder als Bestandteil von Parfümölen. Sie besitzen eine außergewöhnliche Haftfestigkeit und lassen sich sehr gut mit den gebräuchlichen Parfüminhaltsstoffen und anderen Riechstoffen zu neuartigen Kompositionen kombinieren, denen sie ebenfalls eine hohe Haftfestigkeit verleihen. Der Anteil der neuen Benzaldehyde in den Kompositionen beträgt im allgemeinen 1 bis 50 Gew.%. Die Kompositionen können in der Extrait-Parfümerie sowie zur Parfümierung von kosmetischen Präparaten, wie Cremes, Lotionen, Duftwässern, Aerosolen, Seifen, Mundpflegemitteln u.a. verwendet werden. Sie können ferner zur Geruchsverbesserung technischer Produkte, wie Wasch- und Reinigungsmitteln oder Weichspülern verwendet werden.

Beispiel 1

Elektrosynthese von 4-(1-Methoxi-1-methyl-ethyl)-benzaldehyddimethylacetal der Formel:

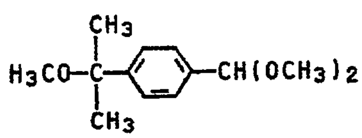

aus 4-(1-Methoxi-1-methyl-ethyl)-toluol.

Apparatur: ungeteilte Zelle mit 5 Elektroden, Elektrodenabstand: 1,5 mm. Anoden: Graphit; Kathoden: Graphit.

In einem ersten Ansatz wird für die Elektrolyse ein Elektrolyt mit folgender Zusammensetzung eingesetzt: 14,03 kg
4-(1-Methoxi-1-methyl-ethyl)-toluol, 0,63 kg $KSO_3C_6H_5$, 0,16 kg $C_6H_5SO_3H$, 62,2 kg $CH_3OH$.

Die Elektrolyse wird mit 11 F/Mol 4-(1-Methoxi-1-methyl-ethyl)-toluol bei einer Stromdichte von 3,6 A/dm² und der Temperatur von 26°C vorgenommen. Dabei wird der Elektrolyt mit 800 l/h über einen Wärmetauscher durch die Zelle gepumpt.

In einem 2. Ansatz wird die Elektrolyse mit folgendem Elektrolyten analog durchgeführt.

Elektrolyt: 11,83 kg 4-(1-Methoxi-1-methyl-ethyl)-toluol 0,68 kg $KSO_3C_6H_5$, 0,17 kg $C_6H_5SO_3H$, 67,7 kg $CH_3OH$.

Die beiden Elektrolyseausträge werden nach Beendigung der Elektrolysen vereinigt und zusammen aufgearbeitet.

Aufarbeitung:

Zunächst wird Methanol bei Normaldruck bis zu Sumpftemperaturen von 125°C abdestilliert. Der Rückstand wird auf ca. 30°C abgekühlt und über eine Drucknutsche filtriert. Hierbei erhält man 0,9 kg eines Salzes, das zusammen mit dem Methanol zur Elektrolyse zurückgeführt werden kann. Das Filtrat (43,3 kg) wird dann bei 2 mbar Kopfdruck und 100 bis 120°C (Kopftemperaturen) rektifiziert. Hierbei erhält man 0,17 kg 4-(1-Methoxi-1-methyl-ethyl)-toluol, 1,54 kg 1-Methoxy-methyl-4-(1-methoxi-1-methylethyl)-benzol und 20,88 kg
4-(1-Methoxi-1-methyl-ethyl)-benzaldehyddimethylacetal. [Kp: 108°C bei 3 mbar; HNMR:

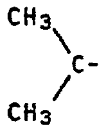 1,53 ppm (s); 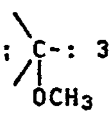 3,05 ppm (s); 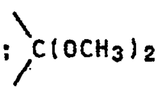 3,35 ppm (s);

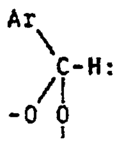 5,38 ppm (s); ArH: 7,44 ppm (s)].

Hieraus errechnen sich ein Umsatz, bezogen auf 4-(1-Methoxi-1-methyl-ethyl)-toluol, von 99,3 %, eine Ausbeute an 1-Methoximethyl-4-(1-methoxi-1-methyl-ethyl)-benzol von 5 % und eine Ausbeute an 4-(1-Methoxi-1-methyl-ethyl)-benzaldehyddimethylacetal von 59,1 %. Die Selektivität für das Dimethylacetal beträgt 62,7 %. Die nicht umgesetzte Ausgangsverbindung und das 1-Methoxymethyl-4-(1-methoxy-1-methyl-ethyl)-benzol können zur Elektrolyse rückgeführt werden.

Beispiel 2

Elektrosynthese von 4-(1-Methoxi-1-methyl-ethyl)-benzaldehyddimethylacetal der Formel

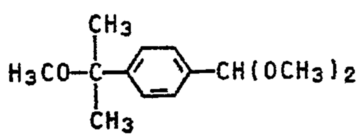

aus 4-Isopropyltoluol.

Apparatur: ungeteilte Zelle mit 11 Elektroden, Elektrodenabstand: 0,5 mm. Anoden: Graphit; Kathoden: Graphit.

Die Elektrolyse wird mit einem Elektrolyten der folgenden Zusammensetzung durchgeführt: 285 g 4-Isopropyltoluol, 30 g $NaSO_3C_6H_5$, 2670 g $CH_3OH$. Die Elektrolyse wird mit 13,5 F/Mol 4-Isopropyltoluol bei einer Stromdichte von 1,7 A/dm$^2$ und einer Temperatur von 35 bis 42°C vorgenommen. Dabei wird der Elektrolyt mit 200 l/h über einen Wärmetauscher durch die Zelle gepumpt.

Aufarbeitung:

Man arbeitet analog Beispiel 1 auf und erhält 154,2 g 4-(1-Methoxi-1-methyl-ethyl)-benzaldehyddimethylacetal. Außerdem werden folgende Verbindungen erhalten, die zur Elektrolyse rückgeführt werden können: 5,9 g 4-Isopropyltoluol, 0,9 g 4-(1-Methoxi-1-methylethyl)-toluol, 1,7 g 1-Methoximethyl-4-isopropyl-benzol, 50,2 g 4-Isopropyl-benzaldehyddimethylacetal und 3,1 g 1-Methoximethyl-4-(1-methoxi-1-methyl-ethyl)benzol.

Hieraus errechnet sich eine Ausbeute an 4-(1-Methoxi-1-methyl-ethyl)-benzaldehyddimethylacetal von 32,4 %, und eine Selektivität für dieses Diacetal von 38,1 %.

Beispiel 3

Elektrosynthese von 4-(1-Methoxi-1-methyl-ethyl)-benzaldehyddimethylacetal der Formel:

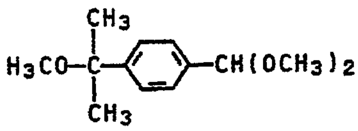

aus 4-Isopropyl-benzaldehyddimethylacetal.

Apparatur: ungeteilte Zelle mit 11 Elektroden, Elektrodenabstand: 1 mm. Anoden: Graphit; Kathoden: Graphit.

Die Elektrolyse wird mit einem Elektrolyten der folgenden Zusammensetzung durchgeführt: 300 g 4-Isopropyl-benzaldehyddimethylacetal, 30 g $KSO_3C_6H_5$, 2670 g $CH_3OH$. Man elektrolysiert mit 12 F/Mol 4-Isopropyl-benzaldehyddimethylacetal bei einer Stromdichte von 3,3 A/dm$^2$ und einer Temperatur von 26 bis 29°C. Dabei wird der Elektrolyt mit 200 l/h über einen Wärmetauscher durch die Zelle gepumpt.

Aufarbeitung:

Man arbeitet analog Beispiel 1 auf und erhält 25 g unumgesetztes 4-Isopropylbenzaldehyddimethylacetal und 72 g 4-(1-Methoxi-1-methyl-ethyl)-benzaldehyddimethylacetal.

Hieraus errechnen sich ein Umsatz, bezogen auf 4-Isopropyl-benzaldehyddimethylacetal von 92 %, eine Ausbeute an 4-(1-Methoxi-1-methyl-ethyl)-benzaldehyddimethylacetal von 21 % und eine Selektivität für 4-(1-Methoxi-1-methyl-ethyl)-benzaldehyddimethylacetal von 23 %.

<u>Beispiel 4</u>

Elektrosynthese von 4-(1-Ethoxi-1-methyl-ethyl)-benzaldehyddiethylacetal der Formel

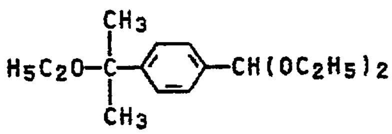

(I),

aus 4-(1-Ethoxi-1-methyl-ethyl)-toluol.

Apparatur: ungeteilte Zelle mit 9 Elektroden, Elektrodenabstand 0,5 mm. Anoden: Graphit; Kathoden: Graphit.

Die Elektrolyse wird mit einem Elektrolyten der folgenden Zusammensetzung durchgeführt:

390 g 4-(1-Ethoxi-1-methyl-ethyl)-toluol, 21,7 g $C_6H_5SO_3H$, 21,7 g $C_6H_5SO_3Na$, 3666 g $C_2H_5OH$.

Man elektrolysiert mit 8,3 F/Mol 4-(1-Ethoxi-1-methyl-ethyl)-toluol bei einer Stromdichte von 1,3 A/dm$^2$ und einer Temperatur von 60°C. Dabei wird der Elektrolyt mit 300 l/h über einen Wärmetauscher durch die Zelle gepumpt.

Aufarbeitung:

Nach Beendigung der Elektrolyse wird der Elektrolyt mit $NaOC_2H_5$ neutralisiert, Ethanol bei Normaldruck und Sumpftemperaturen bis 120°C abdestilliert, das Leitsalz (58 g) abfiltriert und der Rückstand bei 6 mbar Kopfdruck und 85 bis 135°C (Kopftemperaturen) fraktioniert destilliert. Hierbei erhält man 151,4 g 4-(1-Ethoxi-1-methylethyl)-benzaldehyddiethylacetal der oben angegebenen Formel. HNMR: 1,15 bis 1,35 ppm (m), 1,55 ppm (s), 3,25 ppm (q), 3,5 bis 3,7 ppm (m), 5,5 ppm (s), 7,45 ppm (s).

Außerdem werden die folgenden Verbindungen erhalten, die zur Elektrolyse rückgeführt werden können:

5,7 g 4-(1-Ethoxi-1-methyl-ethyl)-toluol und 60,6 g 1-Ethoximethyl-4-(1-ethoxi-1-methyl-ethyl)-benzol.

Hieraus errechnen sich ein Umsatz, bezogen auf 4-(1-Ethoxi-1-methyl-ethyl)-toluol von 98,5 %, eine Ausbeute an 1-Ethoximethyl-4-(1-ethoxi-1-methyl-ethyl)-benzol von 12,5 %, eine Ausbeute an 4-(1-Ethoxi-1-methylethyl)-benzaldehyddiethylacetal von 26,0 % und eine Selektivität für 4-(1-Ethoxi-1-methyl-ethyl)-benzaldehyddiethylacetal von 30,2 %.

<u>Beispiel 5</u>

Synthese von 4-(1-Methoxi-1-methyl-ethyl)-benzaldehyd der Formel

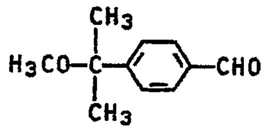

aus 4-(1-Methoxi-1-methyl-ethyl)-benzaldehyddimethylacetal.

In einem Rührkolben werden 1,8 kg 4-(1-Methoxi-1-methyl-ethyl)- benzaldehyddimethylacetal und 1,8 kg Wasser unter Rühren ca. 1 h unter Rückfluß (Sumpftemperatur: 87°C) erhitzt. Danach kühlt man auf ca. 25°C ab, trennt die Phasen und destilliert die organische Phase bei 4 mbar Kopfdruck und 105 bis 108°C (Kopftemperaturen). Hierbei erhält man 1314,6 g 4-(1-Methoxi-1-methyl-ethyl)-benzaldehyd [HNMR: $(CH_3)_2C$-: 1,55 ppm (s), $-OCH_3$: 3,1 ppm (s), ArH: 7,6 ppm (d), 7,90 ppm (d), -CHO: 10,0 ppm (s), $n_D^{25}$: 1,5264].

Dies entspricht einer Ausbeute von 97,3 %.

<u>Beispiel 6</u>

Entsprechend Beispiel 5 erhält man aus 4-(1-Ethoxi-1-methylethyl)-benzaldehyddiethylacetal 4-(1-Ethoxi-1-methyl-ethyl)-benzaldehyd der Formel

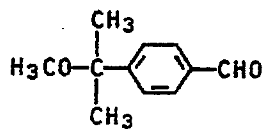

HNMR: 1,2 ppm (t), 1,6 ppm (s), 3,3 ppm (q), 7,65 ppm (d), 7,9 ppm (d), 10,5 ppm (s)

Siedebereich: 269 bis 277°C, $n_D$ : 1.5162

**Patentansprüche**

1. In 4-Stellung durch eine Alkoxialkylgruppe substituierte Benzaldehydderivate der Formel

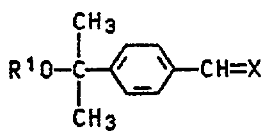

(I),

in der X für ein Sauerstoffatom oder für zwei $R^2O$-Gruppen steht, und $R^1$ und $R^2$ Alkylgruppen mit 1 bis 4 Atomen bedeuten.

2. 4-(1-Alkoxi-1-methylethyl)-benzaldehyde der Formel

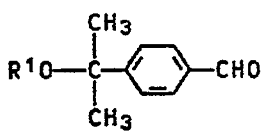

(II)

in der $R^1$ die im Anspruch 1 genannte Bedeutung hat.

3. 4-(1-Alkoxi-1-methylethyl)-benzaldehyddialkylacetale der Formel

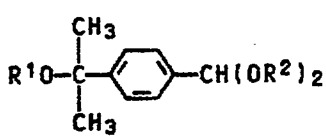

(III),

in der $R^1$ und $R^2$ die im Anspruch 1 genannte Bedeutung haben.

4. 4-(1-Methoxi-1-methyl-ethyl)-benzaldehyddimethylacetal.

5. 4-(1-Methoxi-1-methyl-ethyl)-benzaldehyd.

6. 4-(1-Ethoxi-1-methyl-ethyl)-benzaldehyddiethylacetal.

7. 4-(1-Ethoxi-1-methyl-ethyl)-benzaldehyd.

8. Verfahren zur Herstellung von Acetalen gemäß Anspruch 3, dadurch gekennzeichnet, daß man Benzolderivate der Formel

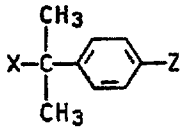 (IV),

in der X ein Wasserstoffatom oder die $R^1O$-Gruppe und Z eine der Gruppen $-CH_3$, $-CH_2OR^2$, $-CH(OR^2)_2$ und $R^1$ und $R^2$ Alkylgruppen mit 1 bis 4 C-Atomen bedeuten, wobei X für ein Wasserstoffatom steht, wenn Z die Gruppe $-CH(OR^2)_2$ bedeutet, in Gegenwart eines Alkanols der Formel ROH, in der R einen Alkylrest mit 1 bis 4 C-Atomen bedeutet, elektrochemisch oxidiert.

9. Verfahren zur Herstellung von Aldehyden gemäß Anspruch 2, dadurch gekennzeichnet, daß man Acetale gemäß Anspruch 3 auf an sich bekannte Weise mit Wasser hydrolysiert.

10. Verwendung der Benzaldehydderivate gemäß Anspruch 1 als Riechstoffe oder als Riechstoffvorprodukte.

## Claims

1. A benzaldehyde derivative which is substituted in the 4-position by alkoxyalkyl and is of the formula

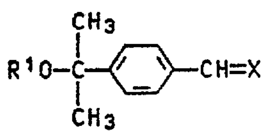 (I),

where X is an oxygen atom or two $R^2O$ groups and $R^1$ and $R^2$ are each alkyl of 1 to 4 carbon atoms.

2. A 4-(1-alkoxy-1-methylethyl)benzaldehyde of the formula

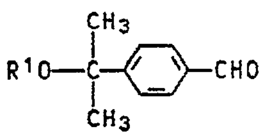 (II)

where $R^1$ has the meaning stated in claim 1.

3. A 4-(1-alkoxy-1-methylethyl)benzaldehyde dialkyl acetal of the formula

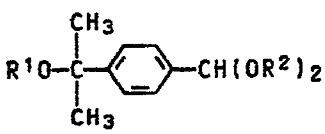 (III),

where $R^1$ and $R^2$ have the meanings stated in claim 1.

4. 4-(1-methoxy-1-methylethyl)benzaldehyde dimethyl acetal.

5. 4-(1-methoxy-1-methylethyl)benzaldehyde.

6. 4-(1-ethoxy-1-methylethyl)benzaldehyde diethyl acetal.

7. 4-(1-ethoxy-1-methylethyl)benzaldehyde.

8. A process for the preparation of an acetal as claimed in claim 3, wherein a benzene derivative of the formula

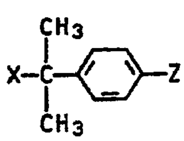 (IV),

where X is hydrogen or an $R^1O$ group and Z is one of the groups $-CH_3$, $-CH_2OR^2$, $-CH(OR^2)_2$ and $R^1$ and $R^2$ are each alkyl of 1 to 4 carbon atoms, X being hydrogen when Z is $-CH(OR^2)_2$, is electrochemically oxidized in the presence of an alkanol of the formula ROH, where R is alkyl of 1 to 4 carbon atoms.

9. A process for the preparation of an aldehyde as claimed in claim 2, wherein an acetal as claimed in claim 3 is hydrolyzed with water in a conventional manner.

10. Use of a benzaldehyde derivative as claimed in claim 1 as a scent or scent intermediate.

## Revendications

1. Dérivés de benzaldéhyde substitués en position 4 par un groupement alcoxyalkyle, de formule

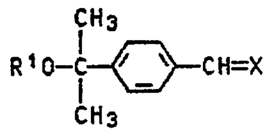

(I),

dans laquelle X est mis pour un atome d'oxygène ou pour deux groupements $R^2O$, et $R^1$ et $R^2$ représentent des groupements alkyle de 1 à 4 atomes de carbone.

2. 4-(1-Alcoxy-1-méthyléthyl)benzaldéhyde de formule

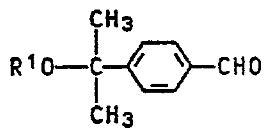

(II)

dans laquelle $R^1$ a la signification donnée dans la revendication 1.

3. 4-(1-Alcoxy-1-méthyléthyl)benzaldéhydedialkylacétals de formule

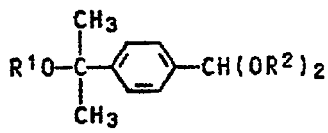

(III),

dans laquelle $R^1$ et $R^2$ ont les significations données dans la revendication 1.

4. 4-(1-Méthoxy-1-méthyléthyl)-benzaldéhydediméthylacétal.

5. 4-(1-Méthoxy-1-méthyléthyl)-benzaldéhyde.

6. 4-(1-Ethoxy-1-méthyléthyl)-benzaldéhydediéthylacétal.

7. 4-(1-Ethoxy-1-méthyléthyl)-benzaldéhyde.

8. Procédé de préparation d'acétals selon la revendication 3, caractérisé en ce qu'on oxyde par voie électrochimique, en présence d'un alcanol de formule ROH où R représente un groupement alkyle de 1 à 4 atomes de carbone, des dérivés de benzène de formule

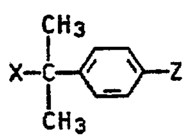

(IV),

dans laquelle X est un atome d'hydrogène ou le groupement $R^1O$ et Z l'un des groupements $-CH_3$, $-CH_2OR^2$, $-CH(OR^2)_2$ et $R^1$ et $R^2$ représentent des groupements alkyle de 1 à 4 atomes de carbone, X étant un atome d'hydrogène quand Z représente le groupement $-CH(OR^2)_2$.

9. Procédé de préparation d'aldéhydes selon la revendication 2, caractérisé en ce qu'on hydrolyse d'une façon connue en soi avec de l'eau les acétals selon la revendication 3.

10. Utilisation des dérivés de benzaldéhyde selon la revendication 1 comme parfums ou comme précurseurs de parfums.